# EUROPEAN PATENT APPLICATION

(11) **EP 4 732 881 A1**
(43) Date of publication of application: **29.04.2026**
(21) Application number: 24825930.1
(22) Date of filing: 19.06.2024
(51) Int. Cl.: A61M 25/10

(54) **BALLOON CATHETER AND METHOD FOR EXPANDING BALLOON OF SAID BALLOON CATHETER**

(30) Priority: 22.06.2023 JP 2023102820; 22.06.2023 JP 2023102821; 22.06.2023 JP 2023102822
(71) Applicant: Kaneka Corporation, Osaka-shi, Osaka 530-8288 (JP)
(72) Inventor: MATSUFUJI, Kazuki, Settsu-shi, Osaka 566-0072 (JP); KOJIMA, Masahiro, Settsu-shi, Osaka 566-0072 (JP); HAMABUCHI, Takahisa, Osaka-shi, Osaka 530-8288 (JP)
(74) Representative: Plasseraud IP
(86) International application number: PCT/JP2024/022200
(87) International publication number: WO 2024/262529

(57) **Abstract**

An object is to provide a balloon catheter having balloons having large diameters and high withstanding pressures, a balloon catheter having balloons easily insertable into a blood vessel, each of the balloons being inflatable to have a large diameter and also having a perfusion function at the time of inflation, and a method for inflating the balloons of the balloon catheters.

A balloon catheter includes a shaft extending in a longitudinal direction from a proximal side to a distal side, a first balloon disposed at a distal part of the shaft, and a balloon group including a plurality of second balloons arranged in a circumferential direction of an outer periphery of the first balloon, wherein during inflation of the first balloon and the balloon group, adjacent ones of the second balloons constituting the balloon group are in contact with each other.

## Description

### TECHNICAL FIELD

The present invention relates to a balloon catheter and a method for inflating balloons of the balloon catheter.

### BACKGROUND ART

Diseases such as angina pectoris and myocardial infarction are caused by the formation of stenosis sites hardened by calcification and other factors in the inner walls of blood vessels. One of the treatment methods for these diseases is angioplasty, in which a balloon catheter is used to dilate the stenosis site. Such angioplasty is sometimes also called Percutaneous Transluminal Angioplasty (PTA) or Percutaneous Transluminal Coronary Angioplasty (PTCA). Angioplasty is a minimally invasive therapy that does not require a thoracotomy such as a bypass surgery, and is widely conducted.

A balloon catheter to be used for angioplasty at least has: a shaft extending in a longitudinal direction from a proximal side to a distal side; a balloon disposed at a distal part of the shaft; and an inflation lumen which is in communication with the balloon and through which a fluid for inflating or deflating the balloon can flow. The balloon is inserted into a blood vessel and delivered to a stenosis site through the inside of the blood vessel in a deflated state. After the balloon is delivered to the stenosis site, a fluid is introduced into or discharged from the inflation lumen using an indeflator (balloon pressurizer) connected to the inflation lumen, thereby controlling inflation and deflation of the balloon.

An example of such a balloon catheter is described in Patent document 1. FIG. 42 of Patent document 1 shows an inflation device including an inner balloon member and a plurality of outer balloon members, and FIG. 43A shows a state where the outer balloon members are inflated. In Patent document 1, a large space is formed between adjacent ones of the outer balloon members to achieve perfusion of blood.

### RELATED ART DOCUMENT

### PATENT DOCUMENT

Patent Document 1: U.S. Patent Application Publication No. 2012/0209375 Specification

### SUMMARY OF THE INVENTION

### PROBLEMS TO BE SOLVED BY THE INVENTION

In the balloon catheter described in Patent document 1, the adjacent outer balloon members are away from each other. Thus, it is considered that, when the outer balloon members are pressed against a stenosis site at the time of dilating the stenosis site, the outer balloon members are displaced in a circumferential direction of the inner balloon member, and the stenosis site cannot be sufficiently dilated. Also, in order to dilate the stenosis site, balloons having large diameters and high withstanding pressures are required. However, if the diameters of balloons at the time of inflation are increased, the diameters of the balloons in a folded state at the time of deflation also increase. Thus, during insertion into a blood vessel, such balloons come into contact with the inner wall of the blood vessel, resulting in deterioration of insertability into the blood vessel.

Meanwhile, a stenosis of a blood vessel occurs also in, for example, the aortic valve. In a case where a stenosis occurs in the aortic valve, the aortic valve is removed, and a new bioprosthetic valve is implanted. However, the implanted bioprosthetic valve degrades over time and needs to be replaced after about 5 to 10 years. In a case where treatment for transcatheter implantation of a new bioprosthetic valve (prosthetic valve) is conducted to deal with degradation of the surgically implanted bioprosthetic valve (prosthetic valve), the area of the valve port sometimes becomes small. Thus, the size of the new bioprosthetic valve needs to be smaller than the bioprosthetic valve having degraded. This decreased size leads to decrease in the flow rate of blood and occurrence of a pressure difference between the front and rear sides of the bioprosthetic valve, resulting in a burden on the heart. In view of this, deformation or rupture of the bioprosthetic valve having been implanted but degraded is considered to enable implantation of a larger bioprosthetic valve. In order to deform or rupture the bioprosthetic valve having degraded, development of balloons having large diameters is required. In addition, development of balloons having high withstanding pressures is required. Moreover, it is desired that, during such a procedure, blood is perfused on the front and rear sides of the balloons.

An object of the present invention is to provide a balloon catheter having balloons having large diameters and high withstanding pressures (hereinafter, sometimes referred to as "first balloon catheter" or "second balloon catheter"). Another object of the present invention is to provide a balloon catheter having balloons easily insertable into a blood vessel, each of the balloons being inflatable to have a large diameter and also having a perfusion function at the time of inflation (hereinafter, sometimes referred to as "third balloon catheter"). Another object of the present invention is to provide a method for inflating the balloons of the balloon catheters.

### SOLUTIONS TO THE PROBLEMS

The first balloon catheter and the method for inflating the balloons according to one or more embodiments of the present invention are configured as follows.
[1-1] A balloon catheter including:
   a shaft extending in a longitudinal direction from a proximal side to a distal side;
   a first balloon disposed at a distal part of the shaft; and
   a balloon group including a plurality of second balloons arranged in a circumferential direction of an outer periphery of the first balloon,
   in which during inflation of the first balloon and the balloon group, adjacent ones of the second balloons constituting the balloon group are in contact with each other.
[1-2] The balloon catheter according to [1-1],
   in which during inflation of the first balloon and the balloon group, the balloon group is in contact with an outer peripheral surface of the first balloon.
[1-3] The balloon catheter according to [1-1] or [1-2],
   in which during inflation of the first balloon and the balloon group, magnitudes of maximum outer diameters of the second balloons constituting the balloon group are all equal to one another and are equal to a magnitude of a maximum outer diameter of the first balloon.
[1-4] The balloon catheter according to [1-1] or [1-2],
   in which during inflation of the balloon group, the second balloons constituting the balloon group are two or more types of second balloons having respective different magnitudes of maximum outer diameters.
[1-5] The balloon catheter according to [1-4],
   in which during inflation of the first balloon and the balloon group, two types of the second balloons having the respective different magnitudes of maximum outer diameters are disposed in the circumferential direction of the outer periphery of the first balloon and include second balloons having a larger maximum outer diameter and a second balloon that has a smaller maximum outer diameter and that is interposed between the second balloons having the larger maximum outer diameter.
[1-6] The balloon catheter according to [1-1] or [1-2],
   in which during inflation of the first balloon and the balloon group, magnitudes of maximum outer diameters of the second balloons constituting the balloon group are all equal to one another and are different from a magnitude of a maximum outer diameter of the first balloon.
[1-7] The balloon catheter according to [1-6],
   in which the magnitudes of the maximum outer diameters of the second balloons constituting the balloon group are smaller than the magnitude of the maximum outer diameter of the first balloon.
[1-8] The balloon catheter according to [1-7],
   in which the shaft has a first inflation lumen and a plurality of second inflation lumens extending in the longitudinal direction of the shaft,
   the first inflation lumen is connected to the first balloon,
   the plurality of second inflation lumens are connected to the plurality of respective second balloons constituting the balloon group,
   the plurality of second inflation lumens are in communication with one proximal second inflation lumen at positions proximal to positions of the connection to the plurality of second balloons, and
   a cross-sectional area S2 in a direction perpendicular to a longitudinal direction of the proximal second inflation lumen is larger than a cross-sectional area S1 in a direction perpendicular to a longitudinal direction of the first inflation lumen.
[1-9] The balloon catheter according to [1-8],
   in which a total cross-sectional area in a direction perpendicular to a longitudinal direction of the plurality of second inflation lumens is larger than the cross-sectional area S1 in the direction perpendicular to the longitudinal direction of the first inflation lumen.
[1-10] The balloon catheter according to [1-8] or [1-9],
   in which a smallest cross-sectional area among cross-sectional areas in a direction perpendicular to a longitudinal direction of the plurality of second inflation lumens is larger than the cross-sectional area S1 in the direction perpendicular to the longitudinal direction of the first inflation lumen.
[1-11] The balloon catheter according to any one of [1-1] to [1-6],
   in which the shaft has a first inflation lumen and a plurality of second inflation lumens extending in the longitudinal direction of the shaft,
   the first inflation lumen is connected to the first balloon, and
   the plurality of second inflation lumens are connected to the plurality of respective second balloons constituting the balloon group.
[1-12] The balloon catheter according to [1-11],
   in which the plurality of second inflation lumens are in communication with one proximal second inflation lumen at positions proximal to positions of the connection to the plurality of second balloons.
[1-13] The balloon catheter according to [1-12],
   in which a cross-sectional area S1 in a direction perpendicular to a longitudinal direction of the first inflation lumen is equal to or larger than a cross-sectional area S2 in a direction perpendicular to a longitudinal direction of the proximal second inflation lumen.
[1-14] The balloon catheter according to [1-13],
   in which the cross-sectional area S1 in the direction perpendicular to the longitudinal direction of the first inflation lumen is equal to or larger than a total cross-sectional area in a direction perpendicular to a longitudinal direction of the plurality of second inflation lumens.
[1-15] The balloon catheter according to [1-13] or [1-14],
   in which the cross-sectional area S1 in the direction perpendicular to the longitudinal direction of the first inflation lumen is equal to or larger than a largest cross-sectional area among cross-sectional areas in a direction perpendicular to a longitudinal direction of the plurality of second inflation lumens.
[1-16] The balloon catheter according to any one of [1-1] to [1-15],
   in which the balloon catheter is used for dilation of an aortic valve, deformation of a bioprosthetic valve implanted in a heart, or rupture of the bioprosthetic valve.
[1-17] A method for inflating the balloons of the balloon catheter according to any one of [1-3] to [1-6], the method including:
   inflating the first balloon by a pressurizer; and
   inflating the balloon group by the pressurizer after the inflation of the first balloon.
[1-18] A method for inflating the balloons of the balloon catheter according to [1-7], the method including:
   inflating the balloon group by a pressurizer; and
   inflating the first balloon by the pressurizer after the inflation of the balloon group.

The second balloon catheter according to one or more embodiments of the present invention is configured as follows.
[2-1] A balloon catheter including:
   a shaft extending in a longitudinal direction from a proximal side to a distal side;
   a first balloon disposed at a distal part of the shaft; and
   a balloon group including a plurality of second balloons arranged in a circumferential direction of an outer periphery of the first balloon,
   in which the second balloons have a withstanding pressure value P2 larger than a withstanding pressure value P1 of the first balloon.
[2-2] The balloon catheter according to [2-1],
   in which a ratio (P2/P1) of the withstanding pressure value P2 to the withstanding pressure value P1 has a value of 1.2 to 16.
[2-3] The balloon catheter according to [2-1] or [2-2],
   in which during inflation of the first balloon and the balloon group, adjacent ones of the second balloons constituting the balloon group are in contact with each other.
[2-4] The balloon catheter according to any one of [2-1] to [2-3],
   in which during inflation of the first balloon and the balloon group, the balloon group is in contact with an outer peripheral surface of the first balloon.
[2-5] The balloon catheter according to any one of [2-1] to [2-4],
   in which during inflation of the first balloon and the balloon group, magnitudes of maximum outer diameters of the second balloons constituting the balloon group are all equal to one another and are equal to a magnitude of a maximum outer diameter of the first balloon.
[2-6] The balloon catheter according to any one of [2-1] to [2-4],
   in which during inflation of the balloon group, the second balloons constituting the balloon group are two or more types of second balloons having respective different magnitudes of maximum outer diameters.
[2-7] The balloon catheter according to [2-6],
   in which during inflation of the first balloon and the balloon group, two types of the second balloons having the respective different magnitudes of maximum outer diameters are disposed in the circumferential direction of the outer periphery of the first balloon and include second balloons having a larger maximum outer diameter and a second balloon that has a smaller maximum outer diameter and that is interposed between the second balloons having the larger maximum outer diameter.
[2-8] The balloon catheter according to any one of [2-1] to [2-4],
   in which during inflation of the first balloon and the balloon group, magnitudes of maximum outer diameters of the second balloons constituting the balloon group are all equal to one another and are different from a magnitude of a maximum outer diameter of the first balloon.
[2-9] The balloon catheter according to [2-8],
   in which the magnitudes of the maximum outer diameters of the second balloons constituting the balloon group are smaller than the magnitude of the maximum outer diameter of the first balloon.
[2-10] The balloon catheter according to [2-9],
   in which the shaft has a first inflation lumen and a plurality of second inflation lumens extending in the longitudinal direction of the shaft,
   the first inflation lumen is connected to the first balloon,
   the plurality of second inflation lumens are connected to the plurality of respective second balloons constituting the balloon group,
   the plurality of second inflation lumens are in communication with one proximal second inflation lumen at positions proximal to positions of the connection to the plurality of second balloons, and
   a cross-sectional area S2 in a direction perpendicular to a longitudinal direction of the proximal second inflation lumen is larger than a cross-sectional area S1 in a direction perpendicular to a longitudinal direction of the first inflation lumen.
[2-11] The balloon catheter according to any one of [2-1] to [2-8],
   in which the shaft has a first inflation lumen and a plurality of second inflation lumens extending in the longitudinal direction of the shaft,
   the first inflation lumen is connected to the first balloon, and
   the plurality of second inflation lumens are connected to the plurality of respective second balloons constituting the balloon group.
[2-12] The balloon catheter according to any one of [2-1] to [2-11],
   in which the balloon catheter is used for dilation of an aortic valve, deformation of a bioprosthetic valve implanted in a heart, or rupture of the bioprosthetic valve.

The third balloon catheter according to one or more embodiments of the present invention is configured as follows.
[3-1] A balloon catheter including:
   a shaft extending in a longitudinal direction from a proximal side to a distal side;
   a first balloon disposed at a distal part of the shaft; and
   a balloon group including a plurality of second balloons arranged in a circumferential direction of an outer periphery of the first balloon,
   in which the first balloon has a withstanding pressure value P1 larger than a withstanding pressure value P2 of the second balloons.
[3-2] The balloon catheter according to [3-1],
   in which a ratio (P2/P1) of the withstanding pressure value P2 to the withstanding pressure value P1 has a value of 0.08 to 0.9.
[3-3] The balloon catheter according to [3-1] or [3-2],
   in which during inflation of the first balloon and the balloon group, adjacent ones of the second balloons constituting the balloon group are in contact with each other.
[3-4] The balloon catheter according to any one of [3-1] to [3-3],
   in which during inflation of the first balloon and the balloon group, the balloon group is in contact with an outer peripheral surface of the first balloon.
[3-5] The balloon catheter according to any one of [3-1] to [3-4],
   in which during inflation of the first balloon and the balloon group, magnitudes of maximum outer diameters of the second balloons constituting the balloon group are all equal to one another and are equal to a magnitude of a maximum outer diameter of the first balloon.
[3-6] The balloon catheter according to any one of [3-1] to [3-4],
   in which during inflation of the balloon group, the second balloons constituting the balloon group are two or more types of second balloons having respective different magnitudes of maximum outer diameters.
[3-7] The balloon catheter according to [3-6],
   in which during inflation of the first balloon and the balloon group, two types of the second balloons having the respective different magnitudes of maximum outer diameters are disposed in the circumferential direction of the outer periphery of the first balloon and include second balloons having a larger maximum outer diameter and a second balloon that has a smaller maximum outer diameter and that is interposed between the second balloons having the larger maximum outer diameter.
[3-8] The balloon catheter according to any one of [3-1] to [3-4],
   in which during inflation of the first balloon and the balloon group, magnitudes of maximum outer diameters of the second balloons constituting the balloon group are all equal to one another and are different from a magnitude of a maximum outer diameter of the first balloon.
[3-9] The balloon catheter according to [3-8],
   in which the magnitudes of the maximum outer diameters of the second balloons constituting the balloon group are smaller than the magnitude of the maximum outer diameter of the first balloon.
[3-10] The balloon catheter according to [3-9],
   in which the shaft has a first inflation lumen and a plurality of second inflation lumens extending in the longitudinal direction of the shaft,
   the first inflation lumen is connected to the first balloon,
   the plurality of second inflation lumens are connected to the plurality of respective second balloons constituting the balloon group,
   the plurality of second inflation lumens are in communication with one proximal second inflation lumen at positions proximal to positions of the connection to the plurality of second balloons, and
   a cross-sectional area S2 in a direction perpendicular to a longitudinal direction of the proximal second inflation lumen is larger than a cross-sectional area S1 in a direction perpendicular to a longitudinal direction of the first inflation lumen.
[3-11] The balloon catheter according to any one of [3-1] to [3-8],
   in which the shaft has a first inflation lumen and a plurality of second inflation lumens extending in the longitudinal direction of the shaft,
   the first inflation lumen is connected to the first balloon, and
   the plurality of second inflation lumens are connected to the plurality of respective second balloons constituting the balloon group.
[3-12] The balloon catheter according to any one of [3-1] to [3-11],
   in which the balloon catheter is used for dilation of an aortic valve, deformation of a bioprosthetic valve implanted in a heart, or rupture of the bioprosthetic valve.

### ADVANTAGEOUS EFFECTS OF THE INVENTION

Since the first balloon catheter has a first balloon and a balloon group including a plurality of second balloons arranged in a circumferential direction of an outer periphery of the first balloon, inflation of both the first balloon and the balloon group enables increase in the outer diameters of the balloons of the balloon catheter including the first balloon and the balloon group. In addition, in the first balloon catheter, since adjacent ones of the second balloons constituting the balloon group are in contact with each other during inflation of the first balloon and the balloon group, the second balloons become less likely to be displaced in the circumferential direction of the outer periphery of the first balloon even when the second balloons are pressed against a stenosis site. As a result, the balloon group can assuredly dilate the stenosis site. In addition, in the first balloon catheter, since adjacent ones of the second balloons constituting the balloon group are in contact with each other during inflation of the first balloon and the balloon group, the second balloons mutually suppress respective inflations even when the pressure for inflating the second balloons is increased. Consequently, a high withstanding pressure is obtained, whereby the hardness (expansive force) of each of the second balloons becomes high.

Since the second balloon catheter has a first balloon and a balloon group including a plurality of second balloons arranged in a circumferential direction of an outer periphery of the first balloon, inflation of both the first balloon and the balloon group enables increase in the outer diameters of the balloons of the balloon catheter including the first balloon and the balloon group. In addition, in the second balloon catheter, since the second balloons have a withstanding pressure value P2 larger than a withstanding pressure value P1 of the first balloon, the second balloons become less likely to be deformed even when stress is concentrated on the balloon group upon inflation of the first balloon and the balloon group. Thus, the withstanding pressures of the second balloons become high, and rupture forces thereof can be increased. In addition, in the second balloon catheter, since the first balloon has a withstanding pressure value P1 smaller than the withstanding pressure value P2 of the second balloons, when the balloon group including the plurality of second balloons is inflated, the first balloon functions as a cushioning material, and the second balloons become less likely to be displaced in the circumferential direction and a radial direction of the first balloon. Thus, a site other than a desired site can be prevented from being dilated, whereby safety becomes high.

Since the third balloon catheter has a first balloon and a balloon group including a plurality of second balloons arranged in a circumferential direction of an outer periphery of the first balloon, inflation of both the first balloon and the balloon group enables increase in the outer diameters of the balloons of the balloon catheter including the first balloon and the balloon group. In addition, in the third balloon catheter, since the first balloon has a withstanding pressure value P1 larger than a withstanding pressure value P2 of the second balloons, it becomes easy for the second balloons to be evenly inflated in the circumferential direction of the outer periphery of the first balloon, with the first balloon being a central axis. In addition, since the second balloons have a withstanding pressure value P2 smaller than the withstanding pressure value P1 of the first balloon, the second balloons are softer than the first balloon. Consequently, the balloon catheter can be inserted into, for example, a blood vessel without damaging the blood vessel, whereby minimal invasiveness can be guaranteed. In addition, since the second balloons have a withstanding pressure value P2 smaller than the withstanding pressure value P1 of the first balloon, when the balloon group is pressed against a stenosis site during inflation of the first balloon and the balloon group, gaps are formed between the balloon group and the stenosis site, whereby the perfusion function is improved.

### BRIEF DESCRIPTION OF THE DRAWINGS

[FIG. 1] FIG. 1 is a side view (partially transparent view) of a balloon catheter.
[FIG. 2] FIG. 2 is a side view (partially transparent view) of balloons of a balloon catheter.
[FIG. 3] FIG. 3 is a cross-sectional view taken along line I-I of balloons shown in FIG. 2.
[FIG. 4] FIG. 4 is a cross-sectional view of balloons different from balloons shown in FIG. 2.
[FIG. 5] FIG. 5 is a cross-sectional view of balloons different from balloons shown in FIG. 2.
[FIG. 6] FIG. 6 is a cross-sectional view in the longitudinal direction of the shaft of the balloon catheter.

### DESCRIPTION OF EMBODIMENTS

The first balloon catheter is characterized in that it has a shaft extending in a longitudinal direction from a proximal side to a distal side, a first balloon disposed at a distal part of the shaft, and a balloon group including a plurality of second balloons arranged in a circumferential direction of an outer periphery of the first balloon, wherein during inflation of the first balloon and the balloon group, adjacent ones of the second balloons constituting the balloon group are in contact with each other. The second balloon catheter is characterized in that it has a shaft extending in a longitudinal direction from a proximal side to a distal side, a first balloon disposed at a distal part of the shaft, and a balloon group including a plurality of second balloons arranged in a circumferential direction of an outer periphery of the first balloon, wherein the second balloons have a withstanding pressure value P2 larger than a withstanding pressure value P1 of the first balloon. The third balloon catheter is characterized in that it has a shaft extending in a longitudinal direction from a proximal side to a distal side, a first balloon disposed at a distal part of the shaft, and a balloon group including a plurality of second balloons arranged in a circumferential direction of an outer periphery of the first balloon, wherein the first balloon has a withstanding pressure value P1 larger than a withstanding pressure value P2 of the second balloons. The following describes the first balloon catheter to the third balloon catheter. In the following description, the first balloon catheter to the third balloon catheter may be collectively referred to simply as "the balloon catheter" or "balloon catheter 1" without distinguishing them.

The following description specifically explains the balloon catheter according to one or more embodiments of the present invention with reference to the drawings. However, the present invention is not limited to the following embodiments. It is obvious that the present invention can be carried out by making modifications in accordance with the gist described above or later, and such modifications are also included in the technical scope of the present invention. In each drawing, hatching, reference signs for components, and the like may be omitted for convenience of description, and in such a case, the specification and other drawings are to be referred to. The dimensions of the various components in the drawings are provided for the purpose of facilitating the understanding of the feature of the present invention, and the dimensions may differ from the actual dimensions in some cases.

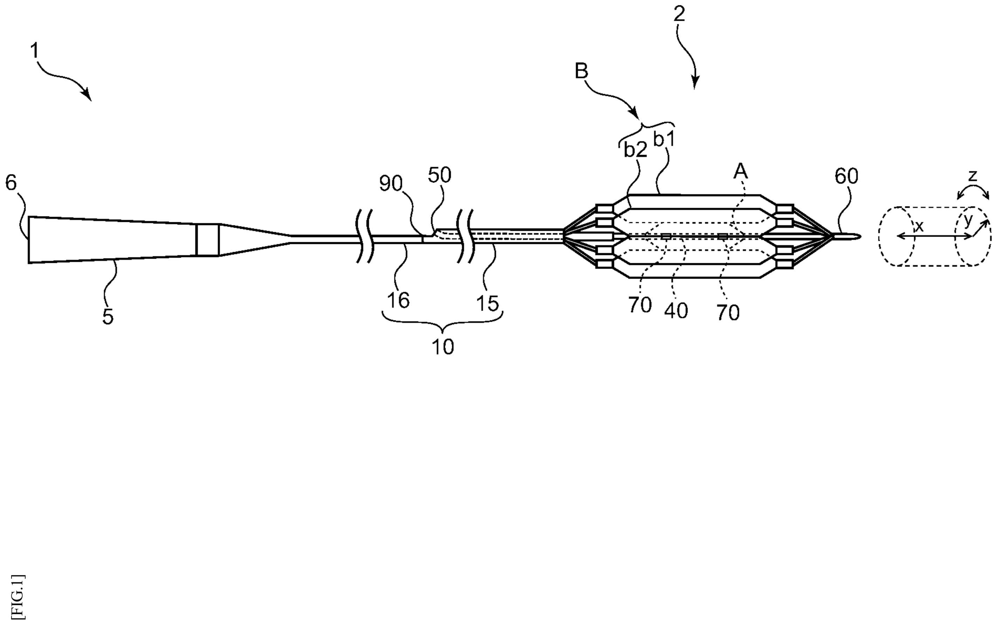

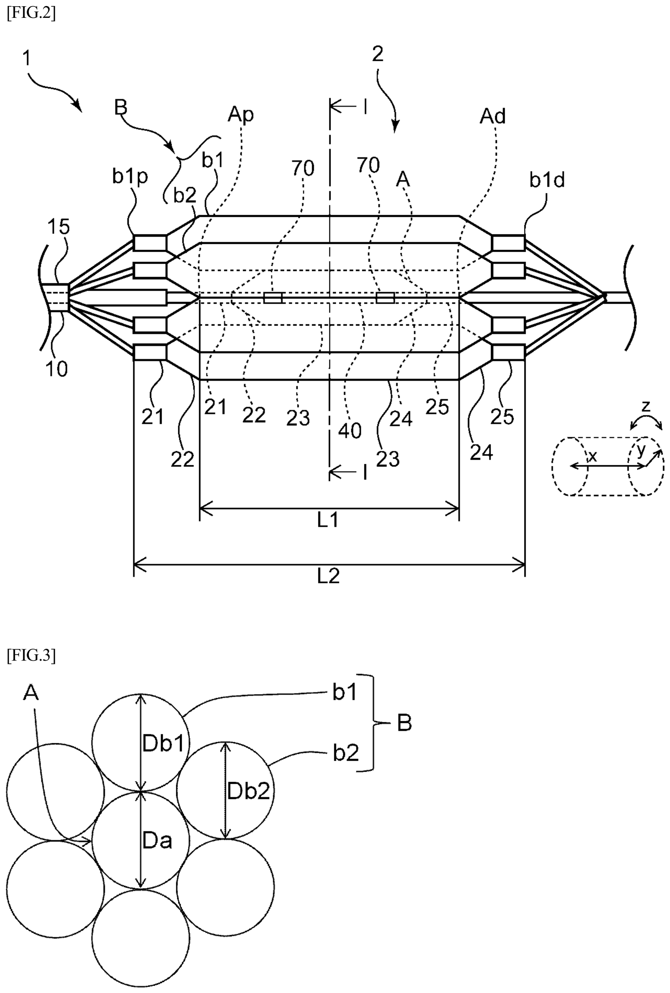

FIG. 1 is a side view (partially transparent view) of the balloon catheter according to one or more embodiments of the present invention. FIG. 2 is a side view (partially transparent view) of balloons of the balloon catheter according to one or more embodiments of the present invention. FIG. 2 shows a state where a first balloon A and a balloon group B including a plurality of second balloons are inflated. FIG. 3 is a cross-sectional view taken along line I-I of the balloons shown in FIG. 2.

As shown in FIG. 1 and FIG. 2, a balloon catheter 1 has: a shaft 10 extending in a longitudinal direction from a proximal side to a distal side; the first balloon A disposed at a distal part of the shaft 10; and the balloon group B including a plurality of second balloons arranged in a circumferential direction of an outer periphery of the first balloon A. Hereinafter, the balloon of the balloon catheter 1 including the first balloon A and the plurality of second balloons constituting the balloon group B may be simply referred to as "balloon 2".

The shaft 10 has: a longitudinal direction x; a radial direction y connecting the centroid of the outer edge of the shaft 10 and any point on the outer edge in a cross section perpendicular to the longitudinal direction x; and a circumferential direction z extending along the outer edge of the shaft 10 in the cross section perpendicular to the longitudinal direction x. In the present description, the hand side of a user in the longitudinal direction x is referred to as "proximal side", and a side opposite to the proximal side, i.e., patient side, is referred to as "distal side". In each of FIG. 1 and FIG. 2, the left side of the drawing is the proximal side (operator side), and the right side of the drawing is the distal side (lesion side).

Each of the members and portions other than the shaft 10 also has a longitudinal direction, a radial direction, and a circumferential direction. These directions may be identical to or different from the longitudinal direction x, the radial direction y, and the circumferential direction z of the shaft 10. However, in the present description, explanations will be given on the assumption that all of the members and portions have a longitudinal direction, a radial direction, and a circumferential direction identical to the longitudinal direction x, the radial direction y, and the circumferential direction z of the shaft 10 in order to facilitate understanding.

The first balloon A and the second balloons are connected to the distal part of the shaft 10. Introduction of a fluid through a lumen of the shaft 10 enables inflation of the first balloon A and the second balloons. Meanwhile, discharge of the introduced fluid enables deflation of the first balloon A and the second balloons. In order to control inflation and deflation of the first balloon A and the second balloons, an indeflator (balloon pressurizer) may be used to introduce or discharge the fluid. As the fluid, for example, a mixture of a contrast medium and a physiological saline is used. The fluid may be a pressurized fluid obtained through pressurization by a pump or the like.

The balloon catheter 1 shown in FIG. 2 and FIG. 3 includes one first balloon A and six second balloons, and the six second balloons constitute a balloon group B. In FIG. 2 and FIG. 3, two second balloons among the six second balloons are denoted by the reference character b1 and the reference character b2 for convenience in explanations. During inflation of the first balloon A and the second balloons b1 and b2, the first balloon A has a maximum outer diameter denoted by Da, and the second balloons b1 and b2 have maximum outer diameters denoted by Db1 and Db2, respectively.

As shown in FIG. 2 and FIG. 3, in the balloon catheter 1, the balloon group B including the plurality of second balloons is arranged in the circumferential direction z of the outer periphery of the first balloon A, and inflation of both the first balloon A and the balloon group B enables increase in the outer diameter of the balloon 2. As a result, the stenosis site can be reliably dilated.

As shown in FIG. 2 and FIG. 3, a first balloon catheter 1 is such that, during inflation of the first balloon A and the balloon group B, the second balloon b1 and the second balloon b2 adjacent to each other among the plurality of second balloons constituting the balloon group B are in contact with each other. Since the second balloon b1 and the second balloon b2 adjacent to each other are in contact with each other, the second balloons mutually suppress respective inflations. Consequently, a high withstanding pressure is obtained, whereby expansive force of the balloon group B can be increased. In addition, since the second balloon b1 and the second balloon b2 adjacent to each other are in contact with each other, the second balloons become less likely to be displaced in the circumferential direction z of the outer periphery of the first balloon A even when the second balloons are brought into contact with a stenosis site. Consequently, the stenosis site can be assuredly dilated. During inflation of the first balloon A and the balloon group B, only at least one pair of adj acent second balloons among the plurality of second balloons constituting the balloon group B have to be in contact with each other, two or more pairs of adjacent second balloons among the plurality of second balloons are preferably in contact with each other, and all of adjacent second balloons among the plurality of second balloons are more preferably in contact with each other. By all of the adjacent second balloons being in contact with each other, the second balloons can be evenly inflated.

In a second balloon catheter 1, the second balloons have a withstanding pressure value P2 larger than a withstanding pressure value P1 of the first balloon A (P2>P 1). Since the second balloons have a withstanding pressure value P2 larger than the withstanding pressure value P1 of the first balloon A, the balloon group B including the plurality of second balloons functions as a reinforcing layer for the first balloon A, and the pressure resistance of the balloon group B is improved. In addition, since the second balloons have a withstanding pressure value P2 larger than the withstanding pressure value P1 of the first balloon A, the second balloons easily bite into a blood vessel, whereby a slip in the longitudinal direction of the shaft 10 during inflation of the balloons can be suppressed. In addition, since the first balloon A has a withstanding pressure value P1 smaller than the withstanding pressure value P2 of the second balloons, when the plurality of second balloons are inflated, the first balloon A functions as a cushioning material, and the second balloons become less likely to be displaced in the radial direction y and the circumferential direction z of the first balloon A. Thus, a site other than a desired site can be prevented from being dilated by the balloon group B, thereby improving safety.

In the second balloon catheter 1, a ratio (P2/P1) of the withstanding pressure value P2 of the second balloons to the withstanding pressure value P1 of the first balloon A only has to have a value of more than 1. The value is preferably from 1.2 to 16. By the value of the ratio (P2/P1) being 1.2 or more, the pressure resistance of the balloon group B becomes high. Consequently, the balloon group B effectively functions as a reinforcing layer for the first balloon A, and the pressure resistance of the balloon group B is increased. The value of the ratio (P2/P1) is more preferably 1.5 or more and further preferably 2 or more. However, when the value of the ratio (P2/P1) is excessively large, the difference between the withstanding pressure values of the first balloon A and the balloon group B becomes excessively large, whereby the first balloon A is squashed by the balloon group B, and the second balloons are displaced in the radial direction y of the first balloon A. Thus, it becomes difficult to evenly inflate the balloon group B. Considering this, the value of the ratio (P2/P1) is preferably 15 or less, more preferably 13 or less, and further preferably 10 or less.

The withstanding pressure value P1 of the first balloon A in the second balloon catheter 1 is, for example, preferably from 4 to 30 atm. This withstanding pressure value P1 is more preferably 6 atm or more and further preferably 8 atm or more, and meanwhile, is more preferably 28 atm or less and further preferably 25 atm or less. The withstanding pressure value P2 of the second balloons in the second balloon catheter 1 is, for example, preferably from 24 to 60 atm. This withstanding pressure value P2 is more preferably 27 atm or more and further preferably 30 atm or more, and meanwhile, is more preferably 50 atm or less and further preferably 40 atm or less.

The withstanding pressure values of the first balloon A and the second balloons in the second balloon catheter 1 can be measured by the following procedure. A water tank with the water temperature adjusted to 37°C±1°C by a heater is prepared, and each of the first balloon A and the second balloons of the balloon catheter 1 is immersed in the water tank. A leak tester is connected to the catheter, a power supply of the heater is turned off, and pressure is continuously applied until the balloon bursts. Then, the pressure at which the balloon has burst is measured, and is regarded as a withstanding pressure value. The pressure application to the balloon is performed under conditions of an initial introduction pressure of 2.0 atm and a rate of 0.2 atm per second. The maximum pressure in the pressure application is set to 40 atm. Such a withstanding pressure value is measured for each of the first balloon A and the second balloons. Regarding the withstanding pressure value P2 of the second balloons, withstanding pressure values of at least three second balloons arbitrarily selected from the plurality of second balloons are measured, and the average value of these withstanding pressure values is regarded as the withstanding pressure value P2 of the second balloons. The withstanding pressure value P2 of the second balloons may be the average value of withstanding pressure values of 10 second balloons or may be the average value of withstanding pressure values of 20 second balloons.

In the second balloon catheter 1, the film thickness of the first balloon A and the film thickness of each of the second balloons may be equal to each other, but the film thickness of the second balloon is preferably larger than the film thickness of the first balloon A. By setting the film thickness of the second balloon to be larger, the withstanding pressure value P2 of the second balloons can be easily made larger than the withstanding pressure value P1 of the first balloon A. The film thickness of the first balloon A and the film thickness of each of the second balloons may be respectively measured at the straight tubular part 23 of the first balloon A and a straight tubular part 23 of the second balloon.

As shown in FIG. 2 and FIG. 3, the second balloon catheter 1 is preferably configured such that, during inflation of the first balloon A and the balloon group B, the adjacent second balloons b1 and b2 among the plurality of second balloons constituting the balloon group B are in contact with each other. Because the adjacent second balloons b1 and b2 are in contact, the second balloons mutually suppress their respective inflations, thereby increasing the withstanding pressure, and consequently the expansive force of the balloon group B can be increased. In addition, by the adjacent second balloons b1 and b2 being in contact with each other, the second balloons become less likely to be displaced in the circumferential direction z of the outer periphery of the first balloon A even when the second balloons are brought into contact with a stenosis site. Consequently, the stenosis site can be assuredly dilated.

During inflation of the first balloon A and the balloon group B in the second balloon catheter 1, only at least one pair of adj acent second balloons among the plurality of second balloons constituting the balloon group B have to be in contact with each other, two or more pairs of adjacent second balloons among the plurality of second balloons are preferably in contact with each other, and all the adjacent second balloons among the plurality of second balloons are more preferably in contact with each other. When all the adjacent second balloons are in contact with each other, the second balloons can be evenly inflated.

In a third balloon catheter 1, the first balloon A has a withstanding pressure value P1 larger than the withstanding pressure value P2 of the second balloons (P1>P2). Since the first balloon A has a withstanding pressure value P1 larger than the withstanding pressure value P2 of the second balloons, it becomes easy for the second balloons to be evenly inflated in the circumferential direction of the outer periphery of the first balloon A, around the outer periphery of the first balloon A serving as a central axis. In addition, since the second balloons have a withstanding pressure value P2 smaller than the withstanding pressure value P1 of the first balloon A, the second balloons are softer than the first balloon A. Consequently, the balloon catheter can be inserted into, for example, a blood vessel without damaging the blood vessel or surrounding tissue. In addition, since the second balloons have a withstanding pressure value P2 smaller than the withstanding pressure value P1 of the first balloon A, when the balloon group B is pressed against a stenosis site during inflation of the first balloon A and the balloon group B, gaps are formed between the balloon group B and the stenosis site, whereby the perfusion function is improved.

In the third balloon catheter 1, the ratio (P2/P1) of the withstanding pressure value P2 of the second balloons to the withstanding pressure value P1 of the first balloon A only has to have a value of less than 1. The value is preferably from 0.03 to 0.8. By the value of the ratio (P2/P1) being 0.8 or less, it becomes easy for the second balloons to be evenly inflated in the circumferential direction of the outer periphery of the first balloon A, with the first balloon A being a central axis. In addition, the insertability of the balloon catheter into a blood vessel and its perfusion function are improved. The value of the ratio (P2/P1) is more preferably 0.7 or less and further preferably 0.6 or less. However, when the value of the ratio (P2/P1) is excessively small, the withstanding pressure value of the second balloons becomes small, whereby the second balloons are easily damaged. Considering this, the value of the ratio (P2/P1) is preferably 0.03 or more, more preferably 0.1 or more, and further preferably 0.2 or more.

The withstanding pressure value P1 of the first balloon A in the third balloon catheter 1 is, for example, preferably from 28 to 60 atm. This withstanding pressure value P1 is more preferably 30 atm or more and further preferably 32 atm or more, and meanwhile, is more preferably 50 atm or less and further preferably 40 atm or less. The withstanding pressure value P2 of the second balloons in the third balloon catheter 1 is, for example, preferably from 2.2 to 45 atm. This withstanding pressure value P2 is more preferably 3 atm or more and further preferably 6 atm or more, and meanwhile, is more preferably 30 atm or less and further preferably 25 atm or less.

The withstanding pressure values of the first balloon A and the second balloons in the third balloon catheter 1 can be measured by the following procedure. A water tank with the water temperature adjusted to 37°C±1°C by a heater is prepared, and each of the first balloon A and the second balloons of the balloon catheter 1 is immersed in the water tank. A leak tester is connected to the catheter, a power supply of the heater is turned off, and pressure is continuously applied until the balloon bursts. Then, the pressure at which the balloon has burst is measured, and is regarded as a withstanding pressure value. The pressure application to the balloon is performed under conditions of an initial introduction pressure of 2.0 atm and a rate of 0.2 atm per second. The maximum pressure in the pressure application is set to 40 atm. Such a withstanding pressure value is measured for each of the first balloon A and the second balloons. Regarding the withstanding pressure value P2 of the second balloons, withstanding pressure values of at least three second balloons arbitrarily selected from the plurality of second balloons are measured, and the average value of these withstanding pressure values is regarded as the withstanding pressure value P2 of the second balloons. The withstanding pressure value P2 of the second balloons may be the average value of withstanding pressure values of 10 second balloons or may be the average value of withstanding pressure values of 20 second balloons.

In the third balloon catheter 1, the film thickness of the first balloon A and the film thickness of each of the second balloons may be equal to each other, but the film thickness of the first balloon A is preferably larger than the film thickness of the second balloon. By setting the film thickness of the first balloon A to be larger, the withstanding pressure value P1 of the first balloon A can be easily made larger than the withstanding pressure value P2 of the second balloons. The film thickness of the first balloon A and the film thickness of each of the second balloons may be respectively measured at the straight tubular part 23 of the first balloon A and the straight tubular part 23 of the respective second balloon.

As shown in FIG. 2 and FIG. 3, the third balloon catheter 1 is preferably configured such that, during inflation of the first balloon A and the balloon group B, the adjacent second balloons b1 and b2 among the plurality of second balloons constituting the balloon group B are in contact with each other. By the adjacent second balloons b1 and b2 being in contact with each other, the second balloons mutually suppress respective inflations. Consequently, a high withstanding pressure is obtained, whereby expansive force of the balloon group B can be increased. In addition, by the adj acent second balloons b1 and b2 being in contact with each other, the second balloons become less likely to be displaced in the circumferential direction z of the outer periphery of the first balloon A even when the second balloons are brought into contact with a stenosis site. Consequently, the stenosis site can be assuredly dilated.

During inflation of the first balloon A and the balloon group B in the third balloon catheter 1, only at least one pair of adjacent second balloons among the plurality of second balloons constituting the balloon group B have to be in contact with each other, two or more pairs of adjacent second balloons among the plurality of second balloons are preferably in contact with each other, and all the adjacent second balloons among the plurality of second balloons are more preferably in contact with each other. When all the adjacent second balloons are in contact with each other, the second balloons can be evenly inflated.

During inflation of the first balloon A and the balloon group B, the second balloons constituting the balloon group B do not have to be in contact with an outer peripheral surface of the first balloon A, but at least one of the plurality of second balloons constituting the balloon group B is preferably in contact with the outer peripheral surface of the first balloon A. By at least one of the plurality of second balloons being in contact with the outer peripheral surface of the first balloon A, the at least one second balloon in contact with the outer peripheral surface of the first balloon A makes it less likely for the second balloons to be displaced in the radial direction y of the first balloon A even when the second balloons are pressed against a stenosis site. Consequently, the stenosis site can be assuredly dilated. All of the plurality of second balloons constituting the balloon group B are more preferably in contact with the outer peripheral surface of the first balloon A. When all of the plurality of second balloons are in contact with the outer peripheral surface of the first balloon A, the first balloon A and the second balloons are mutually inflated even when the second balloons are pressed against a stenosis site. Consequently, the withstanding pressure of the balloon group B can be increased.

The number of the second balloons constituting the balloon group B is preferably three or more, more preferably four or more, and further preferably five or more. By setting the lower limit value of the number of the second balloons constituting the balloon group B to the above range, the balloon group B easily encloses the outer periphery of the first balloon A and easily suppresses inflation of the first balloon A. As a result, when a fluid is introduced into both the first balloon A and the balloon group B, the first balloon A and the balloon group B mutually suppress respective inflations. Consequently, the withstanding pressure of the balloon 2 can be increased, and the hardness of the balloon 2 is increased, whereby expansive force of the balloon 2 can be improved. In addition, since the first balloon A and the balloon group B mutually suppress respective inflations, the balloon 2 becomes less likely to be inflated. Consequently, the balloon 2 is inhibited from being excessively inflated even when a high pressure is applied to the balloon 2. Thus, the balloon 2 is prevented from being inflated to an outer diameter larger than an intended outer diameter, and damage to a lumen inside a living organism such as the aortic valve is decreased, whereby safety can be improved. The upper limit of the number of the second balloons constituting the balloon group B is not particularly limited. However, for example, the number is preferably 20 or less, more preferably 12 or less, further preferably 10 or less, and particularly preferably eight or less. By setting the upper limit value of the number of the second balloons constituting the balloon group B to the above range, the second balloons constituting the balloon group B become less likely to be displaced in the radial direction y and the circumferential direction z of the first balloon A, and it becomes easy for the balloon group B to suppress inflation of the first balloon A.

During inflation of the first balloon A and the balloon group B, a maximum outer diameter of a circumcircle of the balloon 2 is not particularly limited but is, for example, preferably within a range of 5 mm to 60 mm.

The relationship between the maximum outer diameter Da of the first balloon A and the maximum outer diameters of the second balloons constituting the balloon group B is preferably any one of the following (1) to (3), for example.
(1) During inflation of the first balloon A and the balloon group B, magnitudes of the maximum outer diameters of the second balloons constituting the balloon group B are all equal to one another and are equal to a magnitude of the maximum outer diameter Da of the first balloon A.
(2) During inflation of the balloon group B, the second balloons constituting the balloon group B are two or more types of second balloons having respective different magnitudes of maximum outer diameters.
(3) During inflation of the first balloon A and the balloon group B, the magnitudes of the maximum outer diameters of the second balloons constituting the balloon group B are all equal to one another and are different from the magnitude of the maximum outer diameter Da of the first balloon A.

The maximum outer diameter Da of the first balloon A refers to the maximum equivalent circular diameter of the first balloon A in a cross section perpendicular to the longitudinal direction x of the first balloon A. The maximum outer diameter of each second balloon refers to the maximum equivalent circular diameter of the second balloon in a cross section perpendicular to the longitudinal direction x of the second balloon.
(1) will be described with reference to FIG. 3. As shown in FIG. 3, during inflation of the first balloon A and the balloon group B, the magnitudes of the maximum outer diameters of the second balloons constituting the balloon group B are all equal to one another and are equal to the magnitude of the maximum outer diameter Da of the first balloon A. Consequently, balance is easily attained between a force of inflation of the first balloon A and forces of inflation of the second balloons to suppress the inflation of the first balloon A. As a result, the hardnesses of the first balloon A and the balloon group B are increased, the expansive force of the balloon group B can be easily increased, and the stenosis site can be evenly dilated.

In a case where the maximum outer diameters of the first balloon A and all of the second balloons are equal to one another, the maximum outer diameters of the first balloon A and the second balloons are, for example, preferably from 3 mm to 8 mm. The magnitudes of the maximum outer diameters of the plurality of second balloons constituting the balloon group B being all equal to one another means that the maximum outer diameters of the plurality of second balloons constituting the balloon group B are approximately equal to one another, and specifically means that the largest maximum outer diameter among the maximum outer diameters of the second balloons constituting the balloon group B is 100% or more and 110% or less of the smallest maximum outer diameter among the maximum outer diameters of the second balloons constituting the balloon group B. The maximum outer diameter Da of the first balloon A being equal to the maximum outer diameters of the plurality of second balloons constituting the balloon group B means that the maximum outer diameter Da of the first balloon A and the maximum outer diameters of the plurality of second balloons constituting the balloon group B are approximately equal to one another, and specifically means that the maximum outer diameter Da of the first balloon A is 90% or more and 110% or less of a maximum outer diameter (e.g., average value) of the second balloons.

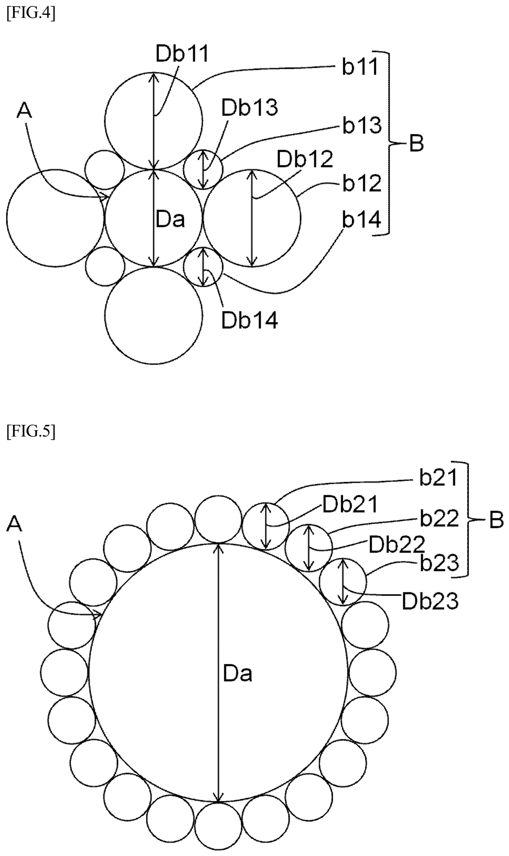

(2) will be described with reference to FIG. 4. FIG. 4 is a cross-sectional view of balloons different from the balloons shown in FIG. 2, at a position corresponding to the position in FIG. 3. As shown in FIG. 4, during inflation of the balloon group B, the second balloons constituting the balloon group B are two or more types of second balloons having respective different magnitudes of maximum outer diameters, whereby the plurality of second balloons having different maximum outer diameters are disposed in the circumferential direction z of the outer periphery of the first balloon A. Consequently, the balloon group B includes: second balloons b11 and b12 that have a larger maximum outer diameter and that are in contact with the inner wall of the blood vessel; and second balloons b13 and b14 that have a smaller maximum outer diameter and that are not in contact with the inner wall of the blood vessel. Thus, spaces are easily formed between the inner wall of the blood vessel and the second balloons b13 and b14 that are not in contact with the inner wall of the blood vessel, whereby blood can be perfused through the spaces. In addition, since the second balloons b11 and b12 having the larger maximum outer diameter in the balloon group B are brought into contact with the inner wall of the blood vessel, the number of the second balloons that are brought into contact with the inner wall of the blood vessel in the balloon group B is limited. Consequently, the number of the contact points between the second balloons and the inner wall of the blood vessel becomes small, whereby stresses to be applied from the second balloons to the inner wall of the blood vessel can be concentrated. As a result, the stenosis site can be assuredly dilated.

In a case where the number of the second balloons constituting the balloon group B is at least three, and, during inflation of the first balloon A and the balloon group B, two types of the second balloons having the respective different magnitudes of maximum outer diameters are disposed in the circumferential direction z of the outer periphery of the first balloon A, the two types of second balloons preferably include the second balloons b11 and b12 having the larger maximum outer diameter and the second balloon b13 that has the smaller maximum outer diameter and that is interposed between the second balloons b11 and b12, as shown in FIG. 4. By such second balloons having the smaller maximum outer diameter being interposed between the second balloons having the larger maximum outer diameter in the circumferential direction z of the outer periphery of the first balloon A, spaces formed between the inner wall of the blood vessel and the second balloons that are not in contact with the inner wall of the blood vessel are present in a distributed manner in the circumferential direction z of the outer periphery of the first balloon A. Consequently, blood can be stably perfused.

During inflation of the balloon group B, the plurality of second balloons constituting the balloon group B may be two types, three types, or four or more types of second balloons having respective different magnitudes of maximum outer diameters, for example. In particular, the second balloons constituting the balloon group B are preferably two types of second balloons having respective different magnitudes of maximum outer diameters.

As shown in FIG. 4, in a case where the plurality of second balloons constituting the balloon group B are two types of second balloons having respective different magnitudes of maximum outer diameters during inflation of the first balloon A and the balloon group B, a ratio (Db11/Db13) of a maximum outer diameter Db11 of the second balloon b11 as the larger maximum outer diameter to a maximum outer diameter Db13 of the second balloon b13 as the smaller maximum outer diameter is, for example, preferably more than 1 and 5 or less. The ratio (Db11/Db13) is more preferably 1.1 or more, further preferably more than 1.1, particularly preferably 2 or more, and most preferably 2.5 or more, and meanwhile, is more preferably 4.5 or less and further preferably 4 or less.

As shown in FIG. 4, in the case where the plurality of second balloons constituting the balloon group B are two types of second balloons having respective different magnitudes of maximum outer diameters during inflation of the first balloon A and the balloon group B, the maximum outer diameter Db13 of the second balloon b13 as the smaller maximum outer diameter is, for example, preferably from 3 mm to 5 mm, and the maximum outer diameter Db11 of the second balloon b11 as the larger maximum outer diameter is, for example, preferably from 3.1 mm to 25 mm (in particular, more than 3.3 mm and 25 mm or less).

(3) will be described with reference to FIG. 5. FIG. 5 is a cross-sectional view of balloons different from the balloons shown in FIG. 2, at the position corresponding to the position in FIG. 3. As shown in FIG. 5, during inflation of the first balloon A and the balloon group B, the magnitudes of the maximum outer diameters of the plurality of second balloons constituting the balloon group B are all equal to one another but are different from the magnitude of the maximum outer diameter Da of the first balloon A. Consequently, a first balloon A and second balloons having different magnitudes of maximum outer diameters can be combined as appropriate to make adjustment, whereby the magnitude of the maximum outer diameter of the balloon group B is easily adjusted. The magnitudes of the maximum outer diameters of the plurality of second balloons constituting the balloon group B being all equal to one another means that the maximum outer diameters of the plurality of second balloons constituting the balloon group B are approximately equal to one another, and specifically means that the largest maximum outer diameter among the maximum outer diameters of the second balloons constituting the balloon group B is 100% or more and 110% or less of the smallest maximum outer diameter among the maximum outer diameters of the second balloons constituting the balloon group B.

In the case of (3), during inflation of the first balloon A and the balloon group B, the magnitudes of the maximum outer diameters of the plurality of second balloons constituting the balloon group B may be larger or smaller than the magnitude of the maximum outer diameter Da of the first balloon A, but are preferably smaller than the magnitude of the maximum outer diameter Da of the first balloon A. By the magnitudes of the maximum outer diameters of the plurality of second balloons constituting the balloon group B being smaller than the magnitude of the maximum outer diameter Da of the first balloon A, the first balloon A can be inflated at a high pressure. Consequently, the stenosis site can be assuredly dilated.

In a case where the magnitudes of the maximum outer diameters of the plurality of second balloons constituting the balloon group B are larger than the magnitude of the maximum outer diameter Da of the first balloon A, a ratio (Db21/Da) of a maximum outer diameter Db21 of a second balloon b21 to the maximum outer diameter Da of the first balloon A is, for example, preferably more than 1 and 4.5 or less. The ratio (Db21/Da) is more preferably 1.1 or more, further preferably more than 1.1, and particularly preferably 1.2 or more, and meanwhile, is more preferably 4 or less and further preferably 3 or less.

In the case where the magnitudes of the maximum outer diameters of the plurality of second balloons constituting the balloon group B are larger than the magnitude of the maximum outer diameter Da of the first balloon A, the maximum outer diameter Da of the first balloon A is, for example, preferably from 3 mm to 5 mm, and the maximum outer diameter Db21 of the second balloon b21 is, for example, preferably from 3.1 mm to 13.5 mm (in particular, more than 3.3 mm and 13.5 mm or less).

In a case where the magnitudes of the maximum outer diameters of the plurality of second balloons constituting the balloon group B are smaller than the magnitude of the maximum outer diameter Da of the first balloon A, a ratio (Db21/Da) of a maximum outer diameter Db21 of a second balloon b21 to the maximum outer diameter Da of the first balloon A is, for example, preferably 0.01 or more and less than 1. The ratio (Db21/Da) is more preferably 0.03 or more, and further preferably 0.05 or more, and meanwhile, is more preferably less than 0.9, further preferably 0.2 or less, and particularly preferably 0.1 or less.

In the case where the magnitudes of the maximum outer diameters of the plurality of second balloons constituting the balloon group B are smaller than the magnitude of the maximum outer diameter Da of the first balloon A, the maximum outer diameter Da of the first balloon A is, for example, preferably from 3 mm to 20 mm, and the maximum outer diameter Db21 of the second balloon b21 is, for example, preferably from 1.0 mm to 5 mm.

In the case of (3), it is preferable that: the shaft 10 has at least a first inflation lumen and a plurality of second inflation lumens extending in the longitudinal direction x of the shaft 10; the first inflation lumen is connected to the first balloon A; and the plurality of second inflation lumens are connected to the plurality of respective second balloons constituting the balloon group B.

By the shaft 10 having the first inflation lumen connected to the first balloon A and the plurality of second inflation lumens connected to the plurality of respective second balloons, a balloon pressurizer connected to the first inflation lumen and a balloon pressurizer connected to each of the plurality of second inflation lumens may be used to introduce fluids to the respective lumens or discharge the fluids from the respective lumens. Consequently, the timings for inflating or deflating the first balloon A and the plurality of second balloons can each be controlled. In addition, the inflation pressures for the first balloon A and the plurality of second balloons can each be controlled. As a result, adaptation to various treatments and situations can be achieved.

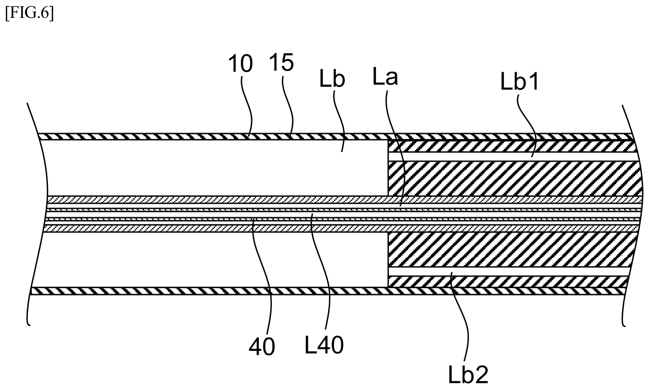

The plurality of second inflation lumens are preferably in communication with one proximal second inflation lumen at positions proximal to positions of the connection to the plurality of second balloons. Such a configuration will be described with reference to FIG. 6. FIG. 6 is a cross-sectional view in the longitudinal direction x of the shaft 10 of the balloon catheter 1. In FIG. 6, the left side of the drawing is the proximal side (operator side), and the right side of the drawing is the distal side (lesion side). As shown in FIG. 6, the shaft 10 has a first inflation lumen La, a second inflation lumen Lb1, and a second inflation lumen Lb2. In FIG. 6, a guidewire tube 40 is disposed in the first inflation lumen La and has a guidewire lumen L40. In a case where the first balloon A (not shown) is connected to the first inflation lumen La, the second balloon b1 (not shown) is connected to the second inflation lumen Lb1, and the second balloon b2 (not shown) is connected to the second inflation lumen Lb2, the second inflation lumen Lb1 and the second inflation lumen Lb2 are preferably in communication with one proximal second inflation lumen Lb at positions proximal to positions of the connection to the second balloons b1 and b2. By the proximal second inflation lumen Lb being in communication with the plurality of second inflation lumens Lb1 and Lb2, a fluid introduced using a balloon pressurizer connected to the proximal second inflation lumen Lb is introduced from the proximal second inflation lumen Lb through the second inflation lumen Lb1 and the second inflation lumen Lb2 into the second balloons b1 and b2, whereby the second balloons b1 and b2 can be simultaneously inflated. Meanwhile, in the case of discharging the fluid from the second balloons b1 and b2, the balloon pressurizer connected to the proximal second inflation lumen Lb may be used to discharge the fluid through the second inflation lumens Lb1 and Lb2, whereby the second balloons b1 and b2 can be simultaneously deflated.

In a case where the plurality of second inflation lumens are in communication with the one proximal second inflation lumen Lb at the positions proximal to the positions of the connection to the plurality of second balloons, a cross-sectional area S2 in a direction perpendicular to the longitudinal direction x of the proximal second inflation lumen Lb is preferably larger than a cross-sectional area S1 in a direction perpendicular to the longitudinal direction x of the first inflation lumen La. By the cross-sectional area S2 being larger than the cross-sectional area S1, the balloon group B is inflated earlier than the first balloon A, whereby the balloon group B can be evenly inflated.

In a case where the cross-sectional area S2 is larger than the cross-sectional area S1, it is preferable that: a total cross-sectional area in a direction perpendicular to the longitudinal direction x of the plurality of second inflation lumens is larger than the cross-sectional area S1; and/or a smallest cross-sectional area among cross-sectional areas in the direction perpendicular to the longitudinal direction x of the plurality of second inflation lumens is larger than the cross-sectional area S1. By the total cross-sectional area in the direction perpendicular to the longitudinal direction x of the plurality of second inflation lumens being larger than the cross-sectional area S1, the balloon group B is inflated earlier than the first balloon A, whereby the balloon group B can be evenly inflated. By the smallest cross-sectional area among the cross-sectional areas in the direction perpendicular to the longitudinal direction x of the plurality of second inflation lumens being larger than the cross-sectional area S1, the balloon group B is inflated earlier than the first balloon A, whereby the balloon group B can be evenly inflated.

In the balloon catheter 1, it is preferable that: the shaft 10 has the first inflation lumen La and the plurality of second inflation lumens extending in the longitudinal direction x of the shaft 10; the first balloon A is connected to the first inflation lumen La; and the second balloons constituting the balloon group B are connected to the plurality of respective second inflation lumens. A pressurizer for introducing or discharging a fluid for inflating or deflating the first balloon A is connected to the first inflation lumen La to which the first balloon A has been connected, and a pressurizer for introducing or discharging a fluid for inflating or deflating the second balloons constituting the balloon group B is connected to each of the second inflation lumens to which the second balloons have been connected. Consequently, the fluids for inflating or deflating the first balloon A and the second balloons can be respectively introduced into or discharged from the first inflation lumen La and the plurality of second inflation lumens at different timings. As a result, the first balloon A and the plurality of second balloons can be inflated or deflated at different timings. For example, the balloon pressurizer connected to the first inflation lumen La is used to introduce a fluid, and then the balloon pressurizer connected to each of the plurality of second inflation lumens is used to introduce a fluid to the lumens, whereby the first balloon A can be inflated first, and then the plurality of second balloons can be inflated. In addition, by connecting a pressurizer to each of the first inflation lumen La and the plurality of second inflation lumens, the inflation pressures for the first balloon A and the plurality of second balloons can each be controlled. As a result, adaptation to various treatments and situations can be achieved.

In a case where the second balloons constituting the balloon group B are connected to the plurality of respective second inflation lumens, the plurality of second inflation lumens are preferably in communication with the one proximal second inflation lumen Lb at the positions proximal to the positions of the connection to the plurality of second balloons. When a pressurizer for introducing or discharging a fluid for inflating or deflating the second balloons is connected to the proximal second inflation lumen Lb, and, for example, the fluid is introduced from the pressurizer, the fluid passes through the proximal second inflation lumen Lb to be introduced into the plurality of second inflation lumens at the same timing. Consequently, the plurality of second balloons can be inflated at the same timing.

In the case where the plurality of second inflation lumens are in communication with the one proximal second inflation lumen Lb at the positions proximal to the positions of the connection to the second balloons, the cross-sectional area S1 in the direction perpendicular to the longitudinal direction x of the first inflation lumen La is preferably equal to or larger than the cross-sectional area S2 in the direction perpendicular to the longitudinal direction x of the proximal second inflation lumen Lb. By the cross-sectional area S1 being equal to the cross-sectional area S2, the plurality of second balloons and the first balloon A can be simultaneously inflated. By the cross-sectional area S1 being larger than the cross-sectional area S2, the first balloon A can be inflated earlier than the plurality of second balloons.

In a case where the cross-sectional area S1 is equal to or larger than the cross-sectional area S2, the cross-sectional area S1 is preferably equal to or larger than the total cross-sectional area in the direction perpendicular to the longitudinal direction x of the plurality of second inflation lumens. By the cross-sectional area S1 being equal to the total cross-sectional area in the direction perpendicular to the longitudinal direction x of the plurality of second inflation lumens, the plurality of second balloons and the first balloon A can be simultaneously inflated. By the cross-sectional area S1 being larger than the total cross-sectional area in the direction perpendicular to the longitudinal direction x of the plurality of second inflation lumens, the first balloon A can be inflated earlier than the plurality of second balloons.

In the case where the cross-sectional area S1 is equal to or larger than the cross-sectional area S2, the cross-sectional area S1 is preferably equal to or larger than a largest cross-sectional area among the cross-sectional areas in the direction perpendicular to the longitudinal direction x of the plurality of second inflation lumens. By the cross-sectional area S1 being equal to the largest cross-sectional area among the cross-sectional areas in the direction perpendicular to the longitudinal direction x of the plurality of second inflation lumens, the plurality of second balloons and the first balloon A can be simultaneously inflated. By the cross-sectional area S1 being larger than the largest cross-sectional area among the cross-sectional areas in the direction perpendicular to the longitudinal direction x of the plurality of second inflation lumens, the first balloon A can be inflated earlier than the plurality of second balloons.

Each of the first balloon A and the second balloons of the balloon catheter 1 preferably has a straight tubular part 23, a proximal tapered part 22 located proximal to the straight tubular part 23, and a distal tapered part 24 located distal to the straight tubular part 23, and may have a proximal sleeve part 21 located proximal to the proximal tapered part 22 and a distal sleeve part 25 located distal to the distal tapered part 24.

During inflation of the first balloon A and the balloon group B, a length L1 of the first balloon A from a distal end Ad thereof to a proximal end Ap thereof in the longitudinal direction x may be equal to a length L2 of the second balloon b1 from a distal end b1d thereof to a proximal end b1p thereof in the longitudinal direction x, but is preferably shorter than the length L2. By the length L1 of the first balloon A being shorter than the length L2 of the second balloon b1, a portion at which the first balloon A is present is easily inflated to a larger extent than a portion at which the first balloon A is not present. As a result, at the portion at which the first balloon A is present, pressure is easily applied, whereby pressure can be applied precisely to a target site. Meanwhile, the portion at which the first balloon A is not present is less likely to be inflated to a large extent, whereby pressure can be made less likely to be applied at this portion. Consequently, a load is less likely to be applied to a site other than the target site, whereby the minimal invasiveness of the balloon catheter 1 can be improved.

During inflation of the first balloon A and the balloon group B, the length L1 of the first balloon A from the distal end Ad thereof to the proximal end Ap thereof in the longitudinal direction x is preferably 95% or less, more preferably 90% or less, and further preferably 85% or less of the length L2 of the second balloon b1 from the distal end b1d thereof to the proximal end b1p thereof in the longitudinal direction x. By setting the upper limit value of the ratio of the length L1 of the first balloon A to the length L2 of the second balloon b1 to the above range, it can be made easy for the balloon catheter 1 to apply a high pressure precisely to the target site. Meanwhile, during inflation of the first balloon A and the balloon group B, the length L1 of the first balloon A from the distal end Ad thereof to the proximal end Ap thereof in the longitudinal direction x is preferably 20% or more, more preferably 25% or more, and further preferably 30% or more of the length L2 of the second balloon b1 from the distal end b1d thereof to the proximal end b1p thereof in the longitudinal direction x. By setting the lower limit value of the ratio of the length L1 of the first balloon A to the length L2 of the second balloon b1 to the above range, it becomes easy for the balloon catheter 1 to apply pressure to a sufficient range of the target site, whereby dilation of the stenosis site, rupture of a bioprosthetic valve, or the like is easily performed.

As shown in FIG. 1, the type of the balloon catheter 1 can be exemplified by a so-called rapid-exchange type balloon catheter having: a guidewire port 50 located midway between the distal and proximal sides of the shaft 10; and a guidewire insertion path extending from the guidewire port 50 to the distal side of the shaft 10.

As shown in FIG. 1, the balloon catheter 1 has a hub 5 on the hand side, and the hub 5 is provided with a fluid inlet 6 into which a fluid for inflating or deflating the balloon 2 is injected.

As shown in FIG. 6, the shaft 10 of the balloon catheter 1 may further have the guidewire tube 40 extending in the longitudinal direction x. As shown in FIG. 1, a portion of the guidewire tube 40 is preferably disposed in a lumen of the first balloon A.

Examples of a material forming the shaft 10 include polyamide-based resins, polyester-based resins, polyurethane-based resins, polyolefin-based resins, fluorine-based resins, vinyl chloride-based resins, silicone-based resins, and natural rubbers. These types of materials may be used singly, or two or more of these types of materials may be used in combination. Among these materials, at least one material selected from polyamide-based resins, polyolefin-based resins, and fluorine-based resins is preferable as the material forming the shaft 10. By the material being any of polyamide-based resins, polyolefin-based resins, or fluorine-based resins, the slipperiness of the surface of the shaft 10 becomes high, whereby the insertability of the balloon catheter 1 in a blood vessel can be improved.

In the case of a rapid-exchange type balloon catheter such as one shown in FIG. 1, the shaft 10 of the balloon catheter 1 preferably has a distal shaft 15 and a proximal shaft 16 disposed proximal to the distal shaft 15, and the distal shaft 15 and the proximal shaft 16 may be separate members. In a case where the distal shaft 15 and the proximal shaft 16 are separate members, the proximal shaft 16 may be made from a resin or a metal.

In the case of a rapid-exchange type balloon catheter such as one shown in FIG. 1, the proximal shaft 16 and/or the distal shaft 15 preferably has an outer wall provided with a coating, and each of both the proximal shaft 16 and the distal shaft 15 is more preferably provided with a coating. The coating may be a hydrophilic coating or a hydrophobic coating according to purpose and can be provided by: immersing the shaft 10 in a hydrophilic coating agent or a hydrophobic coating agent; applying the hydrophilic coating agent or the hydrophobic coating agent onto the outer wall of the shaft 10; or coating the outer wall of the shaft 10 with the hydrophilic coating agent or the hydrophobic coating agent. Each of the coating agents may contain a drug and an additive.

Examples of the hydrophilic coating agent include: hydrophilic polymers such as polyvinyl alcohol, polyethylene glycol, polyacrylamide, polyvinylpyrrolidone, and methyl vinyl ether-maleic anhydride copolymers; and hydrophilic coating agents made by any combination of these hydrophilic polymers. Examples of the hydrophobic coating agent include polytetrafluoroethylene (PTFE), fluorinated ethylene propylene (FEP), perfluoroalkoxy alkane (PFA), silicone oil, hydrophobic urethane resins, carbon coats, diamond coats, diamond-like carbon (DLC) coats, ceramic coats, and substances with low surface free energy terminated with an alkyl group or a perfluoro alkyl group.

The type of the balloon catheter 1 may be a so-called over-the-wire type balloon catheter (not shown) having a guidewire insertion path extending from the distal side to the proximal side of the shaft. In the case of the over-the-wire type balloon catheter, the inflation lumens and the guidewire lumen preferably extend to the hub 5 disposed on the hand side, and each of the lumens preferably has a proximal opening provided in the hub 5 having a bifurcated structure. In the case of the over-the-wire type balloon catheter, an outer shaft preferably has an outer wall provided with a coating. For a material forming the shaft and a coating thereon, description of the rapid-exchange type balloon catheter may be referred to.

Examples of a material forming the first balloon A and materials forming the second balloons include: polyolefin-based resins such as polyethylene, polypropylene, and ethylene-propylene copolymers; polyester-based resins such as polyethylene terephthalate and polyester elastomers; polyurethane-based resins such as polyurethane and polyurethane elastomers; polyphenylene sulfidebased resins; polyamide-based resins such as polyamides and polyamide elastomers; fluorine-based resins; silicone-based resins; and natural rubbers such as latex rubber. These types of materials may be used singly, or two or more of these types of materials may be used in combination. Among these materials, at least one material selected from polyamide-based resins, polyester-based resins, and polyurethane-based resins is preferable as each of the material forming the first balloon A and the materials forming the second balloons.

As each of the material forming the first balloon A and the materials forming the second balloons, an elastomer resin is preferably used from the viewpoint of film thinning and flexibility. For example, among the polyamide-based resins, nylon 12, nylon 11, or the like is suitable as each of the material forming the first balloon A and the materials forming the second balloons. Nylon 12 is more suitable since nylon 12 can be comparatively easily molded during blow molding. From the viewpoint of film thinning and flexibilities of the first balloon A and the second balloons, any of polyamide elastomers such as polyether ester amide elastomers and polyamide ether elastomers is preferably used. Among these polyamide elastomers, any of polyether ester amide elastomers is preferably used since polyether ester amide elastomers have high yield strengths and allow the first balloon A and the second balloons to have favorable dimensional stabilities.

The material forming the first balloon A and the materials forming the second balloons may be identical to each other but are preferably different from each other. In the case of the first balloon catheter and the second balloon catheter, the materials are preferably selected such that the withstanding pressure value P2 of the second balloons becomes larger than the withstanding pressure value P1 of the first balloon A. In the case of the third balloon catheter, the materials are preferably selected such that the withstanding pressure value P1 of the first balloon A becomes larger than the withstanding pressure value P2 of the second balloons. The materials forming the plurality of second balloons may be different from one another but are preferably identical to one another. By the materials being identical to one another, the extents of inflation, the hardnesses, and the like of the individual second balloons can be set to be approximately equal to one another.

The shaft 10 disposed inside the first balloon A has a portion corresponding to the position of the first balloon A in the longitudinal direction x. On this portion, a radiopaque marker 70 may be disposed such that the position of the first balloon A can be ascertained radiographically. The radiopaque marker 70 is preferably disposed at each of positions corresponding to both ends of the straight tubular part 23 of the first balloon A and may be disposed at a position corresponding to the center of the straight tubular part 23 of the first balloon A. The shape of the radiopaque marker 70 is preferably a tubular shape. Examples of the tubular shape include a cylindrical shape, a polygonal tubular shape, a shape obtained by forming a slit in a tube so as to have a C-shaped cross section, and a coil shape obtained by winding a wire material. As a material forming the radiopaque marker 70, for example, a radiopaque substance such as lead, barium, iodine, tungsten, gold, platinum, iridium, stainless steel, titanium, or a cobalt-chromium alloy may be used.

The balloon catheter 1 is preferably provided with a tip member 60 at a distal end part thereof. The tip member 60 may be provided to the distal end part of the balloon catheter 1 by being connected to a distal end part of the first balloon A as a member separate from the shaft 10, or a distal end part of the shaft 10 may function as the tip member 60 by the shaft 10 extending to a position distal to the distal end of the first balloon A.

The balloon catheter 1 is usable for, for example, dilation of a blood vessel. In addition, the balloon catheter 1 is particularly suitably usable for dilation of an aortic valve, deformation of a bioprosthetic valve implanted in a heart, or rupture of the bioprosthetic valve. By deforming or rupturing a bioprosthetic valve implanted in a heart using the balloon catheter 1, a larger bioprosthetic valve can be implanted, and the pressure difference between the front and rear sides of the bioprosthetic valve can be enhanced.

Next, a method for inflating the balloon 2 of the balloon catheter 1 (in particular, first balloon catheter) will be described.

The following case is assumed. That is, the balloon catheter 1 has: the shaft 10 extending in the longitudinal direction x from the proximal side to the distal side; the first balloon A disposed at the distal part of the shaft 10; and the balloon group B including the plurality of second balloons arranged in the circumferential direction z of the outer periphery of the first balloon A. During inflation of the first balloon A and the balloon group B, adjacent ones of the second balloons constituting the balloon group B are in contact with each other. Furthermore, during the inflation, (a) the magnitudes of the maximum outer diameters of the second balloons constituting the balloon group B are all equal to one another and are equal to the magnitude of the maximum outer diameter Da of the first balloon A, (b) the second balloons constituting the balloon group B are two or more types of second balloons having respective different magnitudes of maximum outer diameters, or (c) the magnitudes of the maximum outer diameters of the second balloons constituting the balloon group B are all equal to one another and are different from the magnitude of the maximum outer diameter Da of the first balloon A. In this case, the method preferably includes: inflating the first balloon A by a pressurizer; and inflating the balloon group B by the pressurizer after the inflation of the first balloon A. By inflating the first balloon A earlier than the second balloons, the second balloons are inflated along the outer peripheral surface of the first balloon A, whereby the second balloons are inflated without being displaced in the radial direction y of the first balloon A.

The following case is assumed. That is, the balloon catheter 1 (in particular, first balloon catheter) has: the shaft 10 extending in the longitudinal direction x from the proximal side to the distal side; the first balloon A disposed at the distal part of the shaft 10; and the balloon group B including the plurality of second balloons arranged in the circumferential direction z of the outer periphery of the first balloon A. During inflation of the first balloon A and the balloon group B, adjacent ones of the second balloons constituting the balloon group B are in contact with each other. Furthermore, during the inflation, the magnitudes of the maximum outer diameters of the second balloons constituting the balloon group B are all equal to one another and are different from the magnitude of the maximum outer diameter Da of the first balloon A, and the magnitudes of the maximum outer diameters of the second balloons constituting the balloon group B are smaller than the magnitude of the maximum outer diameter Da of the first balloon A. In this case, the method preferably includes: inflating the balloon group B by a pressurizer; and inflating the first balloon A by the pressurizer after the inflation of the balloon group B. By inflating the second balloons earlier than the first balloon A, the second balloons can be evenly inflated.

This application claims the benefit of the priority date of Japanese patent application No. 2023-102820 filed on June 22, 2023, Japanese patent application No. 2023-102821 filed on June 22, 2023, and Japanese patent application No. 2023-102822 filed on June 22, 2023. All of the contents of the Japanese patent application No. 2023-102820, the Japanese patent application No. 2023-102821, and the Japanese patent application No. 2023-102822 are incorporated by reference herein.

### REFERENCE SIGNS LIST

1 Balloon catheter
2 Balloon
5 Hub
6 Fluid inlet
10 Shaft
15 Distal shaft
16 Proximal shaft
21 Proximal sleeve part
22 Proximal tapered part
23 Straight tubular part
24 Distal tapered part
25 Distal sleeve part
40 Guidewire tube
50 Guidewire port
60 Tip member
70 Radiopaque marker
90 Boundary line between the distal shaft and the proximal shaft
A First balloon
B Balloon group
b1, b2, b11 to b14, and b21 to b23 Second balloon
Da Maximum outer diameter of the first balloon
Db1, Db2, Db11 to Db14, and Db21 to Db23 Maximum outer diameters of the second balloon
L40 Guidewire lumen
La First inflation lumen
Lb Proximal second inflation lumens
Lb1, Lb2 Second inflation lumens

## Claims

1. A balloon catheter comprising:
a shaft extending in a longitudinal direction from a proximal side to a distal side;
a first balloon disposed at a distal part of the shaft; and
a balloon group including a plurality of second balloons arranged in a circumferential direction of an outer periphery of the first balloon,
wherein during inflation of the first balloon and the balloon group, adjacent ones of the second balloons constituting the balloon group are in contact with each other.

2. A balloon catheter comprising:
a shaft extending in a longitudinal direction from a proximal side to a distal side;
a first balloon disposed at a distal part of the shaft; and
a balloon group including a plurality of second balloons arranged in a circumferential direction of an outer periphery of the first balloon,
wherein the second balloons have a withstanding pressure value P2 larger than a withstanding pressure value P1 of the first balloon.

3. The balloon catheter according to claim 2,
wherein a ratio (P2/P1) of the withstanding pressure value P2 to the withstanding pressure value P1 has a value of 1.2 to 16.

4. A balloon catheter comprising:
a shaft extending in a longitudinal direction from a proximal side to a distal side;
a first balloon disposed at a distal part of the shaft; and
a balloon group including a plurality of second balloons arranged in a circumferential direction of an outer periphery of the first balloon,
wherein the first balloon has a withstanding pressure value P1 larger than a withstanding pressure value P2 of the second balloons.

5. The balloon catheter according to claim 4,
wherein a ratio (P2/P1) of the withstanding pressure value P2 to the withstanding pressure value P1 has a value of 0.08 to 0.9.

6. The balloon catheter according to claim 2 or 4,
wherein during inflation of the first balloon and the balloon group, adjacent ones of the second balloons constituting the balloon group are in contact with each other.

7. The balloon catheter according to claim 1, 2, or 4,
wherein during inflation of the first balloon and the balloon group, the balloon group is in contact with an outer peripheral surface of the first balloon.

8. The balloon catheter according to claim 1, 2, or 4,
wherein during inflation of the first balloon and the balloon group, magnitudes of maximum outer diameters of the second balloons constituting the balloon group are all equal to one another and are equal to a magnitude of a maximum outer diameter of the first balloon.

9. The balloon catheter according to claim 1, 2, or 4,
wherein during inflation of the balloon group, the second balloons constituting the balloon group are two or more types of second balloons having respective different magnitudes of maximum outer diameters.

10. The balloon catheter according to claim 9,
wherein during inflation of the first balloon and the balloon group, two types of the second balloons having the respective different magnitudes of maximum outer diameters are disposed in the circumferential direction of the outer periphery of the first balloon and include second balloons having a larger maximum outer diameter and a second balloon that has a smaller maximum outer diameter and that is interposed between the second balloons having the larger maximum outer diameter.

11. The balloon catheter according to claim 1, 2, or 4,
wherein during inflation of the first balloon and the balloon group, magnitudes of maximum outer diameters of the second balloons constituting the balloon group are all equal to one another and are different from a magnitude of a maximum outer diameter of the first balloon.

12. The balloon catheter according to claim 11,
wherein the magnitudes of the maximum outer diameters of the second balloons constituting the balloon group are smaller than the magnitude of the maximum outer diameter of the first balloon.

13. The balloon catheter according to claim 12,
wherein the shaft has a first inflation lumen and a plurality of second inflation lumens extending in the longitudinal direction of the shaft,
the first inflation lumen is connected to the first balloon,
the plurality of second inflation lumens are connected to the plurality of respective second balloons constituting the balloon group,
the plurality of second inflation lumens are in communication with one proximal second inflation lumen at positions proximal to positions of the connection to the plurality of second balloons, and
a cross-sectional area S2 in a direction perpendicular to a longitudinal direction of the proximal second inflation lumen is larger than a cross-sectional area S1 in a direction perpendicular to a longitudinal direction of the first inflation lumen.

14. The balloon catheter according to claim 13,
wherein a total cross-sectional area in a direction perpendicular to a longitudinal direction of the plurality of second inflation lumens is larger than the cross-sectional area S1 in the direction perpendicular to the longitudinal direction of the first inflation lumen.

15. The balloon catheter according to claim 13,
wherein a smallest cross-sectional area among cross-sectional areas in a direction perpendicular to a longitudinal direction of the plurality of second inflation lumens is larger than the cross-sectional area S1 in the direction perpendicular to the longitudinal direction of the first inflation lumen.

16. The balloon catheter according to claim 1, 2, or 4,
wherein the shaft has a first inflation lumen and a plurality of second inflation lumens extending in the longitudinal direction of the shaft,
the first inflation lumen is connected to the first balloon, and
the plurality of second inflation lumens are connected to the plurality of respective second balloons constituting the balloon group.

17. The balloon catheter according to claim 16,
wherein the plurality of second inflation lumens are in communication with one proximal second inflation lumen at positions proximal to positions of the connection to the plurality of second balloons.

18. The balloon catheter according to claim 17,
wherein a cross-sectional area S1 in a direction perpendicular to a longitudinal direction of the first inflation lumen is equal to or larger than a cross-sectional area S2 in a direction perpendicular to a longitudinal direction of the proximal second inflation lumen.

19. The balloon catheter according to claim 18,
wherein the cross-sectional area S1 in the direction perpendicular to the longitudinal direction of the first inflation lumen is equal to or larger than a total cross-sectional area in a direction perpendicular to a longitudinal direction of the plurality of second inflation lumens.

20. The balloon catheter according to claim 18,
wherein the cross-sectional area S1 in the direction perpendicular to the longitudinal direction of the first inflation lumen is equal to or larger than a largest cross-sectional area among cross-sectional areas in a direction perpendicular to a longitudinal direction of the plurality of second inflation lumens.

21. The balloon catheter according to claim 1, 2, or 4,
wherein the balloon catheter is used for dilation of an aortic valve, deformation of a bioprosthetic valve implanted in a heart, or rupture of the bioprosthetic valve.

22. A method for inflating the balloons of the balloon catheter according to claim 8, the method comprising:
inflating the first balloon by a pressurizer; and
inflating the balloon group by the pressurizer after the inflation of the first balloon.

23. A method for inflating the balloons of the balloon catheter according to claim 9, the method comprising:
inflating the first balloon by a pressurizer; and
inflating the balloon group by the pressurizer after the inflation of the first balloon.

24. A method for inflating the balloons of the balloon catheter according to claim 11, the method comprising:
inflating the first balloon by a pressurizer; and
inflating the balloon group by the pressurizer after the inflation of the first balloon.

25. A method for inflating the balloons of the balloon catheter according to claim 12, the method comprising:
inflating the balloon group by a pressurizer; and
inflating the first balloon by the pressurizer after the inflation of the balloon group.
